(19) Europäisches Patentamt
European Patent Office
Office européen des brevets

(11) **EP 2 863 098 B1**

(12) **EUROPÄISCHE PATENTSCHRIFT**

(45) Veröffentlichungstag und Bekanntmachung des Hinweises auf die Patenterteilung:
**19.12.2018 Patentblatt 2018/51**

(51) Int Cl.:
***F16L 11/04*** *(2006.01)*    ***A61L 29/12*** *(2006.01)*

(21) Anmeldenummer: **14187805.8**

(22) Anmeldetag: **06.10.2014**

(54) **Hochdruck-Schlauch mit mehreren Koextrusions-Schichten**

High pressure hose with multiple co-extrusion layers

Tuyau haute pression doté de plusieurs couches de co-extrusion

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.10.2013 DE 102013221101**

(43) Veröffentlichungstag der Anmeldung:
**22.04.2015 Patentblatt 2015/17**

(73) Patentinhaber: **RAUMEDIC AG**
**95213 Münchberg (DE)**

(72) Erfinder: **Grzeskowiak, Jörg**
**95028 Hof (DE)**

(74) Vertreter: **Rau, Schneck & Hübner**
**Patentanwälte Rechtsanwälte PartGmbB**
**Königstraße 2**
**90402 Nürnberg (DE)**

(56) Entgegenhaltungen:
**WO-A1-96/20741        WO-A1-2005/056097**
**US-A1- 2009 017 247**

**Beschreibung**

Die Erfindung betrifft einen Hochdruck-Schlauch mit mehreren Koextrusions-Schichten, insbesondere zur medizinischen Anwendung.

Ein Hochdruck-Schlauch zur Anwendung in der Angiographie ist beispielsweise aus der EP 1 272 238 B1 und aus der EP 1 204 436 B1 bekannt. Weiterhin sind als Hochdruck-Schläuche einerseits koextrudierte Schläuche mit hoher Biegesteifigkeit und andererseits armierte Katheterschläuche mit geringerer Biegesteifigkeit bekannt. Armierte Schläuche, also Schläuche mit Faserarmierungen, sind in der Herstellung teuer. Die US 2009/0 017 247 A1 beschreibt einen Druckluftschlauch aus Polyamid, insbesondere zur Anwendung als Bremsschlauch. Die US 5,624,617 beschreibt ein Verfahren zur Herstellung eines Führungskatheters. Die EP 1 501 562 B1 beschreibt einen Katheterschlauch. Die US 6,520,952 B1 beschreibt einen keramikverstärkten Katheter. Die DE 10 206 062 187 A1 beschreibt ein Katheterrohrelement. Die WO 2010/078 102 A1 beschreibt einen Hochdruck-Infusionskatheter.

Die WO 2005/056 097 A1 offenbart einen koextrudierten medizinischen Schlauch mit mindestens zwei Schichten, von denen eine als Nanocompositematerial ausgeführt ist. Die WO 96/20741 A1 beschreibt einen Mehrschicht-Katatherschlauch.

Es ist eine Aufgabe der vorliegenden Erfindung, einen Hochdruck-Schlauch mit mehreren Koextrusions-Schichten derart weiterzubilden, dass dessen Biegesteifigkeit verringert ist.

Diese Aufgabe ist erfindungsgemäß gelöst durch einen Hochdruck-Schlauch mit den im Anspruch 1 angegebenen Merkmalen.

Erfindungsgemäß wurde erkannt, dass bei Einsatz einer Mischung aus einem Block-Copolymer und einem Homopolymer für die Armierungs-Innenschicht eine deutliche Reduzierung der Biegesteifigkeit des Hochdruck-Schlauchs erreicht werden kann. Ein derartiger Hochdruck-Schlauch, der beispielsweise einem Berstdruck im Bereich von 80 bar standhalten muss, kommt in der Angiographie zum Einsatz.

Eine Innere der Koextrusions-Schichten ist dabei zur Gewährleistung einer Hochdruckbeständigkeit ausgeführt. Eine Äußere der Koextrusions-Schichten ist aus einem besonders biokompatiblen Material. Der Schlauch kann auch mehr als zwei Koextrusions-Schichten aufweisen, z.B. drei, vier oder auch noch mehr Koextrusions-Schichten.

Das Mischungsverhältnis aus 60 bis 90 Teilen Block-Copolymer und 40 bis 10 Teilen Homopolymer ergibt eine starke, erwünschte Herabsetzung der Biegesteifigkeit der Innenschicht und damit des gesamten Schlauchs, wobei trotz des geringen Anteils des Homopolymers eine ausreichende Hochdruckbeständigkeit erhalten bleibt. Das Mischungsverhältnis kann beispielsweise 60:40, 65:35, 70:30, 75:25, 80:20, 85:15 oder auch 90:10 betragen.

Der Hochdruck-Schlauch ist insbesondere insgesamt aus bioverträglichen Materialien gefertigt.

Ein Block-Copolymer mit den Merkmalen der Ansprüche 2 und 3 sowie ein Homopolymer mit den Merkmalen der Ansprüche 4 und 5 haben sich als besonders geeignete Materialbestandteile für die Armierungs-Innenschicht herausgestellt.

Eine Außenschicht mit den Merkmalen der Ansprüche 6 und 7 hat sich für den Schlauch ebenfalls als besonders geeignet herausgestellt. Auch ein anderes Material als Polyurethan für die Außenschicht ist möglich. Die Außenschicht kann aus einem weniger biegesteifen Material gefertigt sein als die Innenschicht und kann eine Shore-Härte von 75A aufweisen.

Ein Schichtstärken-Verhältnis nach Anspruch 8 ergibt eine vorteilhafte Verteilung der Funktionen der Außenschicht einerseits und der Innenschicht andererseits. Die vergleichsweise geringe Schichtstärke der Innenschicht ermöglicht eine weitere Reduzierung der Biegesteifigkeit.

Es hat sich herausgestellt, dass eine relativ geringe Schichtstärke der Innenschicht ausreicht, um einem für einen Hochdruckschlauch vorgegebenen Berstdruck standzuhalten. Das Schichtstärken-Verhältnis I/A kann höchstens 60%, höchstens 55% oder auch höchstens 50% betragen und kann sogar noch kleiner sein, z. B. höchstens 45% oder höchstens 40%.

Die erfindungsgemässe Biegesteifigkeit von kleiner als 3000 mN führt zu einem flexiblen Schlauch, der auch bei Patientenbewegungen wenig oder gar nicht stört. Der Schlauch kann vom Arzt oder der Krankenschwester einfacher angebracht werden.

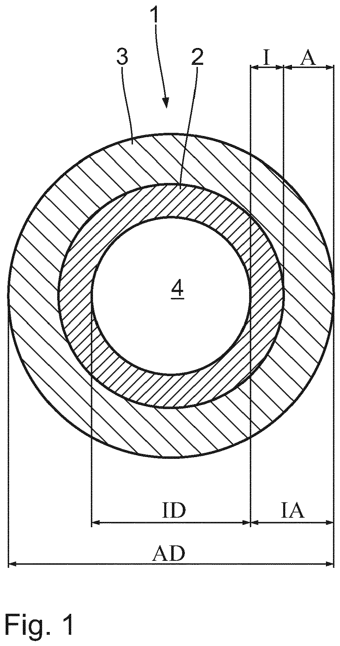

Ein Ausführungsbeispiel der Erfindung wird nachfolgend anhand der Zeichnungen näher erläutert. Die einzige Fig. 1 zeigt einen Querschnitt durch eine Ausführung eines Hochdruck-Schlauchs.

Ein Hochdruck- Schlauch 1 hat mehrere Koextrusions-Schichten. In der Fig. 1 ist der Schlauch 1 zweischichtig dargestellt. Die innere Koextrusions-Schicht ist als Armierungs-Innenschicht 2 ausgeführt. Die äußere Koextrusions-Schicht, also die Außenschicht 3, ist als Polyurethanschicht ausgeführt.

Die Innenschicht 2 weist eine Mischung aus einem Block-Copolymer und einem Homopolymer auf.

Das Block-Copolymer ist ein Polyether-Block-Amid. Das Block-Copolymer hat eine Shore-Härte von 60D bis 66D. Das Block-Copolymer stellt in der Polymermischung der Innenschicht einen die Biegesteifigkeit verringernden Bestandteil dar. Polyether-Anteile des Polyether-Block-Amids dienen dabei zur Verringerung der Biegesteifigkeit.

Das Homopolymer der Innenschicht 2 ist ein Polyamid, nämlich reines PA11. Das Homopolymer hat eine Shore-Härte von 69D bis 75D.

Das Homopolymer sorgt dafür, eine Druckbeständigkeit des Schlauchs 1 zu gewährleisten.

Der Schlauch 1 ist bis zu einem Berstdruck von 83 bar druckstabil. Der Hochdruck-Schlauch 1 hält auch einem Berstdruck von mehr als 90 bar und insbesondere von 95 bar stand.

Die Polymermischung der Innenschicht 2 ist gebildet durch ein Mischungsverhältnis aus 60 bis 90 Teilen Block-Copolymer einerseits und 40 bis 10 Teilen Homopolymer andererseits.

Die Außenschicht 3 hat eine Shore-Härte von 70A bis 76A.

Der Schlauch 1 hat insgesamt einen Außendurchmesser AD im Bereich zwischen 3,61 mm und 3,75 mm. Der dargestellte Schlauch 1 hat einen Außendurchmesser AD von 3,68 mm.

Der Außendurchmesser AD stellt gleichzeitig den Außendurchmesser der Außenschicht 3 dar.

Ein freies Lumen 4 des Schlauchs 1 hat einen Durchmesser ID im Bereich zwischen 1,78 mm und 1,88 mm. Die dargestellte Ausführung hat einen Innendurchmesser von 1,83 mm.

Der Durchmesser ID ist gleichzeitig der Innendurchmesser der Innenschicht 2. Eine gesamte Schichtstärke IA der Koextrusions-Schichten 2,3 des Schlauchs 1 liegt im Bereich zwischen 0,865 mm und 1,43 mm. Die dargestellte Ausführung hat eine gesamte Schichtstärke IA von 0,925 mm.

Die Innenschicht 2 hat eine Schichtstärke I im Bereich zwischen 0,28 mm und 0,33 mm. Die dargestellte Ausführung hat eine Schichtstärke I der Innenschicht 2 von 0,305 mm. Die Außenschicht 3 hat eine Schichtstärke A im Bereich zwischen 0,535 mm und 1,15 mm. Die dargestellte Ausführung hat eine Schichtstärke A der Außenschicht 3 von 0,62 mm.

Ein Verhältnis I/A aus der Schichtstärke I der Innenschicht 2 und der Schichtstärke A der Außenschicht 3 liegt im Bereich zwischen 25% und 65%. Das Verhältnis I/A kann höchstens 60%, höchstens 55% oder auch höchstens 50% betragen. Das Verhältnis I/A kann sogar noch kleiner sein und z.B. höchstens 45% oder höchstens 40% betragen. Das Verhältnis I/A beträgt zur Realisierung eines Berstdrucks von 83 bar mindestens 25%.

Grundsätzlich kann das Verhältnis I/A aber auch noch kleiner sein und beispielsweise 10% betragen.

Der Schlauch 1 hat eine Biegesteifigkeit, die kleiner ist als 3000mN. Diese Biegesteifigkeit wird gemessen über eine zu biegende Länge des Schlauchs 1 von 20,0 mm. Dieser wird mit einer Biegegeschwindigkeit von 6° Biegewinkel pro Sekunde bis zu einem maximalen Biegewinkel von 30° gebogen. Der die Biegesteifigkeit repräsentierende Kraftwert wird nach einer Haltezeit bei diesem Biegewinkel von 30° von 2 Sekunden gemessen.

Die dargestellte und beschriebene Ausführung hat eine Biegesteifigkeit, die sogar kleiner ist 2500mN und die auch kleiner ist als 2200mN.

## Patentansprüche

1. Hochdruck-Schlauch (1) mit mehreren Koextrusions-Schichten

   - wobei eine der Koextrusions-Schichten als Armierungs-Innenschicht (2) ausgeführt ist,
   - wobei die Armierungs-Innenschicht (2) eine Mischung aus einem Block-Copolymer und einem Homopolymer aufweist,
   - mit einem Mischungsverhältnis aus 60 bis 90 Teilen Block-Copolymer und 40 bis 10 Teilen Homopolymer,
   - wobei das Block-Copolymer in der Polymermischung der Innenschicht (2) einen die Biegesteifigkeit des Hochdruck-Schlauchs (1) verringernden Bestandteil darstellt,
   - wobei der Hochdruck-Schlauch (1) eine Biegesteifigkeit aufweist, die kleiner ist als 3000 mN.

2. Schlauch nach Anspruch 1, **dadurch gekennzeichnet, dass** das Block-Copolymer ein Polyether-Block-Amid ist.

3. Schlauch nach Anspruch 1 oder 2, **dadurch gekennzeichnet, dass** Block-Copolymer eine Shore-Härte im Bereich zwischen 60D und 66D aufweist.

4. Schlauch nach einem der Ansprüche 1 bis 3, **dadurch gekennzeichnet, dass** das Homopolymer ein Polyamid ist.

5. Schlauch nach einem der Ansprüche 1 bis 4, **dadurch gekennzeichnet, dass** das Homopolymer eine Shore-Härte im Bereich zwischen 69D bis 75D aufweist.

6. Schlauch nach einem der Ansprüche 1 bis 5, **dadurch gekennzeichnet, dass** eine weitere der Koextrusions-Schichten, die als Außenschicht (3) ausgeführt ist, als Polyurethanschicht ausgeführt ist.

7. Schlauch nach Anspruch 6, **dadurch gekennzeichnet, dass** die Polyurethanschicht eine Shore-Härte von 70A bis 76A aufweist.

8. Schlauch nach einem der Ansprüche 1 bis 7, **gekennzeichnet durch** ein Verhältnis I/A aus einer Schichtstärke I der Innenschicht (2) und einer Schichtstärke A der Außenschicht (3), für das gilt:

$$10\% < I/A < 65\%.$$

## Claims

1. Flexible high-pressure tube (1) with a plurality of coextruded layers

   - where one of the coextruded layers is configured as internal reinforcement layer (2),
- where the internal reinforcement layer (2) comprises a mixture of a block copolymer with a homopolymer,
- with a mixing ratio of from 60 to 90 parts of block copolymer to from 40 to 10 parts of homopolymer,
- where the block copolymer in the polymer mixture of the internal layer (2) is a constituent that reduces the flexural stiffness of the flexible high-pressure tube (1),
- where the flexural stiffness of the flexible high-pressure tube (1) is less than 3000 mN.

2. Flexible tube according to Claim 1, **characterized in that** the block copolymer is a polyether-block-amide.

3. Flexible tube according to Claim 1 or 2, **characterized in that** the Shore hardness of the block copolymer is in the range from 60D to 66D.

4. Flexible tube according to any of Claims 1 to 3, **characterized in that** the homopolymer is a polyamide.

5. Flexible tube according to any of Claims 1 to 4, **characterized in that** the Shore hardness of the homopolymer is in the range from 69D to 75D.

6. Flexible tube according to any of Claims 1 to 5, **characterized in that** another of the coextruded layers, configured as external layer (3), is configured as polyurethane layer.

7. Flexible tube according to Claim 6, **characterized in that** the Shore hardness of the polyurethane layer is from 70A to 76A.

8. Flexible tube according to any of Claims 1 to 7, **characterized by** compliance with 10% < I/A < 65% in respect of the ratio I/A of the layer thickness I of the internal layer (2) to the layer thickness A of the external layer (3).

**Revendications**

1. Tuyau haute pression (1) comportant plusieurs couches de coextrusion

- une des couches de coextrusion étant réalisée comme couche interne d'armature (2)
- la couche interne d'armature (2) comportant un mélange d'un copolymère à blocs et d'un homopolymère
- avec un rapport de mélange de 60 à 90 parties de copolymère à blocs pour 40 à 10 parties d'homopolymère

- le copolymère à blocs dans le mélange de polymères de la couche interne (2) comportant un composant réduisant la rigidité en flexion du tuyau haute pression (1),
- le tuyau haute pression (1) présentant une rigidité en flexion inférieure à 3 000 mN.

2. Tuyau selon la revendication 1, **caractérisé en ce que** le copolymère à blocs est un polyéther bloc amide.

3. Tuyau selon la revendication 1 ou 2, **caractérisé en ce que** le copolymère bloc présente une dureté Shore dans la gamme entre 60D et 66D.

4. Tuyau selon l'une des revendications 1 à 3, **caractérisé en ce que** l'homopolymère est un polyamide.

5. Tuyau selon l'une des revendications 1 à 4, **caractérisé en ce que** l'homopolymère présente une dureté Shore dans la gamme entre 69D et 75D.

6. Tuyau selon l'une des revendications 1 à 5, **caractérisé en ce qu'**une autre des couches de coextrusion, réalisée comme couche externe (3), est réalisée sous forme d'une couche de polyuréthane.

7. Tuyau selon la revendication 6, **caractérisé en ce que** la couche de polyuréthane présente une dureté Shore de 70A à 76A.

8. Tuyau selon l'une des revendications 1 à 7, **caractérisé par** un rapport I/A de l'épaisseur I de la couche interne (2) et de l'épaisseur A de la couche externe (3), pour lequel :

$$10\ \% < I/A < 65\ \%.$$

Fig. 1

**EP 2 863 098 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1272238 B1
- EP 1204436 B1
- US 20090017247 A1
- US 5624617 A
- EP 1501562 B1
- US 6520952 B1
- DE 10206062187 A1
- WO 2010078102 A1
- WO 2005056097 A1
- WO 9620741 A1